# EUROPEAN PATENT APPLICATION

(11) **EP 1 530 970 A1**
(43) Date of publication of application: **18.05.2005**
(21) Application number: 04026883.1
(22) Date of filing: 12.11.2004
(51) Int. Cl.: A61K 35/54, A61P 37/06

(54) **Composition and method for inducing immune tolerance towards cell, tissue and/or organ transplants**

(30) Priority: 13.11.2003 US 519269 P
(71) Applicant: Medizinische Hochschule Hannover, 30625 Hannover (DE)
(72) Inventor: Haverich, Axel Prof. Dr., 30916 Isemhagen (DE); Martin, Ulrich PD Dr., 31319 Sehnde (DE); Fändrich, Fred PD Dr., 24105 Kiel (DE)
(74) Representative: Kröncke, Rolf, Dr.

(57) **Abstract**

The present invention relates to a composition comprising embryonic stem cells or haematopoietic stem cells or precursor cells being obtained by differentiation of said embryonic stem cells as well as trophoblasts, throphoblasts precursor and/or placental cell derived from said trophopblasts, all cell types being derived from the same embryonic stem cell origin. Further, the present invention relates to the induction of immune tolerance in an individual as well as to the use of the compositions according to the present invention in methods for cell, tissue and organ transplantation. The composition according to the present invention may be used in the treatment or therapy of transplantation or gene therapy or in the field of tissue engineering.

## Description

The invention relates to a composition containing two different types of cells both being derived from embryonic stem cell lines and its use in cell, tissue or organ transplantation. In particular, the invention relates to the induction of immune tolerance by using a composition containing trophoblasts derived from embryonic stem cell lines with the help of differentiating factors and embryonic stem cells or haematopoietic stem cells or precursor cells derived therefrom of the same origin in an individual undergoing cell, tissue or organ transplantation of a cell, tissue or organ transplant derived from embryonic stem cell lines of the same origin. Moreover, the invention relates to methods for inducing tolerance in individuals receiving cell, tissue or organ transplants derived from embryonic stem cell lines as well as to the use of the composition in the field of transplantation.

### Prior Art

New technologies of cell transplantation and tissue engineering will help to overcome current therapeutical limits and may enable improved treatment of yet poorly treatable diseases. The use of stem cells in combination with tissue engineering technologies may allow the development of bioartificial tissues and organs. Thus, the employment of human stem cells offers novel therapeutic perspectives for a variety of different diseases. Human ES-cells are considered as excellently suited for regenerative medicine due to their almost unlimited potential for proliferation and differentiation. Since the knowledge on stem cell biology is very limited at present, many problems have to be resolved when using embryonic stem cells in therapy, in order to finally reach the above goals: This includes the development of improved protocols allowing specific differentiation and selection of the resulting cell types. Further, in view of tissue engineering applications, it is still necessary to greatly improve the understanding of how organs normally develop in the fetus, how they acquire structure and function and how to develop better ways to achieve appropriate vascularisation, innervation if required and growth in three dimensions. Moreover, in cases where no autologous adult stem cells can be used, the immunogenicity of the cell transplants is another unresolved problem. The same is true for the clinical use of existing (and e.g. NIH-registered) human embryonic stem cell lines which is hampered by the fact that the transplantation of allogeneic embryonic stem cell derived cells may require life-long application of immunosuppression.

In contrast to adult stem and progenitor cells, embryonic stem cells (ES-cells) unquestionably have the potential to form all types of differentiated and functional cells within the body. Embryonic stem cells can be isolated from the inner cell mass of blastocystes. Pluripotent murine embryonic stem cells were isolated more than 20 years ago (Evans, M.J., and M.H. Kaufman. 1981. *Nature* 292, no. 5819:154). *In vitro* differentiation of murine ES-cells has been investigated extensively. The generation of chimaeras by injection of ES cells into blastocysts (a routine procedure to produce mice carrying mutations in specific genes) demonstrated that ES cells contribute to all cell types of the body (Bradley, A., M. Evans, M.H. Kaufman, and E. Robertson. 1984. *Nature* 309, no. 5965:255.). Therefore, ES cells have been designated pluripotent. In view of very recent results on the potential for differentiation towards trophoblasts or oocytes (Xu, R.H., X. Chen, D.S. Li, R. Li, G.C. Addicks, C. Glennon, T.P. Zwaka, and J.A. Thomson. 2002. *Nat Biotechnol* 20, no. 12:1261; Hubner, K., G. Fuhrmann, L.K. Christenson, J. Kehler, R. Reinbold, R. De La Fuente, J. Wood, J.F. Strauss, 3rd, M. Boiani, and H.R. Scholer. 2003. Science 300, no. 5623:1251), it is currently discussed, whether ES-cells are even totipotent. Unlike murine ES-cells, ES-cells of other species were much more difficult to isolate. In 1995 ES-cells of rhesus monkeys have been isolated while human embryonic stem cells were firstly isolated in 1998. Up until now, probably more than 100 human embryonic cell lines have been isolated world wide, 78 of which have been included in the NIH registry.

Thus, embryonic stem cells are one of the favoured cell types for future clinical applications of cell transplantation and tissue engineering. The distinguishing feature of ES cells is their capacity to be maintained indefinitely in an undifferentiated state in culture (Gepstein, L. 2002. *Circ Res* 91, no. 10:866), thus, forming an embryonic cell line of primary embryonic cells. Whereas embryonic stem cells are in many fields superior compared to adult stem cells in view of the potential for differentiation, there is one major disadvantage of ES-cells. For example, differentiated cells and engineered tissues derived from existing human ES-cell lines would not be isogeneic to the patient. In analogy to organ allotransplantation, long term grafting of such allogeneic cells would require pharmacological immunosuppression. Although immunosuppressive drugs and protocols have been greatly improved during the last decades, improvement in early graft survival has not been achieved without significant infectious morbidity and non-immune side effects. Initial survival has also failed to translate into better long-term graft survival and chronic rejection has continued to recall grafts after the first year at a rate that has been essentially unchanged for the last 20 years. Moreover, late morbidity and mortality from the continued need for non-specific immune suppression has become increasingly evident with longer follow-up (e.g. Peddi, V.R., J. Whiting, P.D. Weiskittel, J.W. Alexander, and M.R. First. 1998. *Am J Kidney Dis* 32, no. 1:101).

In contrast to the transplantation of whole parenchymal organs, there may be several options to allow transplantation of ES-cell-derived cells without need for pharmacological immunosuppression:

In principle, autologous ES-cells can be produced for each patient by means of therapeutic cloning. Besides enormous costs for each application, ethical concerns argue against this approach, as at least one human embryo per patient would have to be produced and destroyed to permit isolation of autologous stem cells. Moreover, procedures of somatic nuclear transfer frequently result in abnormalities due to epigenetic changes.

Another option is the organisation of an ES-cell banking. A collection of several thousand ES-cell lines may guarantee the availability of matched ES-cells not for all but at least for the majority of patients. However, the construction of such cell collections would require destroying thousands of surplus human embryos. The probability of finding a match depends on several factors, including the size of the donor pool, the frequency of the recipient's HLA haplotype and the accepted degree of mismatch. Assuming that a match is only required for HLA-A, HLA-B and HLA-DR antigens and that limited (one or two) mismatches are allowed, an individual is very likely to find a match even among a few thousand donors. A match of that type would nevertheless require immunosuppressive treatment for the rest of the recipient's life. It is unclear whether ES-cell based cell and tissue replacement therapies could be based on partial mismatches combined with immunosuppression. As some of the therapeutic targets (juvenile diabetes, for example) imply lifelong successful engraftment (with the possibility of repeated treatment) it may be advantageous to significantly reduce or eliminate the mismatch between the recipient and the donor ES cells. Such requirement would significantly diminish the probability for any given individual to find a match even with a very large ES cell bank (Beatty, P.G., K.M. Boucher, M. Mori, and E.L. Milford. 2000. *Hum Immunol* 61, no. 8:834).

The feasibility of another option, the development of an universal ES-cell line with non-immunogenic characteristics, is discussed controversially.

A better solution of the problem may be ES-cell mediated tolerance induction: From the transplant immunologist's point of view, tolerance can be defined as the durable persistence of a tissue, in the absence of a detrimental immune response, achieved without ongoing therapeutic intervention. In this context, it is important to note that tolerance is an acquired phenomenon and not an innate property of an individual (Owen, R.D. 1945. *Science* 102:400; Billingham, R.E., L. Brent, and P.B. Medawar. 1953. *Nature* 172:603). Noteworthy, the tolerant state established at birth is continuously restructured as new antigens appear throughout life and our immune system must be able to tolerize these "foreign" antigens such as physiological hormones released in puberty and pregnancy (Goodnow, C.C. 1996. *Proc Natl Acad Sci U S A* 93, no. 6:2264). The fact that fetal life can develop and survive in a MHC-mismatched host is a good example for nature's capacity to discriminate not only between self and non-self but as well between dangerous and non-dangerous (Vacchio, M.S., and S.P. Jiang. 1999. *Crit Rev Immunol* 19, no. 5-6:461).

Fändrich et al. describes an induction of immune tolerance in rats after transplantation of primary embryonic stem cells isolated from blastocysts (Fandrich et al., 2002, Nat. Med. 8(2), 171). It is demonstrated therein that intraportal injection of blastocyste-derived crude cultures of rat ES-like-cells into fully MHC-mismatched rats results in permanent engraftment without supplementary host conditioning. The resulting mixed chimerism was the basis for long-term acceptance of heart allografts isogeneic to the transplanted ES-like-cells. The Graft-derived cells comprised B-cells, T-cells and monocytes.

In view of the above problems regarding the use of embryonic stem cells in cell, tissue and organ transplantation, the object of the present invention is to provide compositions, their use and methods for inducing tolerance in an individual, subsequently undergoing cell, tissue and/or organ transplantation comprising cells, tissue and/or organs derived from embryonic stem cell lines.

Another object is the provision of a composition inducing tolerance in an individual as well as its use in the induction of immune tolerance against transplants derived from embryonic stem cell lines or having the same origin as the cells comprised in the composition.

### Description of the present invention

The invention relates to a composition comprising
a. trophoblasts, precursors of trophoblasts and/or placental cells derived from trophoblasts, whereby said cells are derived from an embryonic stem cell line by differentiation;
b. embryonic stem cells, haematopoietic cells or haematopoietic precursor cells derived from the same embryonic stem cell line used above in a.

Preferably, the composition further contains a pharmaceutically acceptable carrier, additive, diluent and/or additional pharmaceutically active ingredient.

In addition, the present invention relates to the use of said composition for the preparation of a composition for the pre-treatment of individuals receiving cell, tissue and/or organ transplantation, the transplant being derived from an embryonic stem cell line, in order to induce immune tolerance towards the transplant in the individual. The two types of cells, i.e. a) trophoblasts, precursor of trophoblasts and/or placental cells derived from trophoblasts whereby the above cells are derived from the same embryonic stem cell line used for the transplant by differentiation, and b) embryonic stem cells, haematopoietic cells or haematopoietic precursor cells derived from the same embryonic stem cell line used for the transplant may be contained in a kit in a form to be administered to an individual. The kit may further comprise instructions for inducing tolerance in an individual.

That is, the present invention may be used in a method for inducing immune tolerance in an individual receiving a transplant derived from an embryonic stem cell line, comprising the steps of administering a composition according to the present invention comprising
a. trophoblasts, precursors of trophoblasts and/or placental cells derived from trophoblasts, whereby said cells are derived from the same embryonic stem cell line used for the transplant, by differentiation;
b. embryonic stem cells, haematopoietic cells or haematopoietic precursor cells derived from the same embryonic stem cell line used for the transplant;
before receiving the transplant.

Moreover, according to the present invention the composition may be used in a method for embryonic stem cell line derived cell, tissue and/or organ transplantation comprising administering the composition according to the present invention comprising
a) trophoblasts, precursors of trophoblasts and/or placental cells derived from trophoblasts, whereby said cells are derived by differentiation from the same embryonic stem cell line used for the transplant;
b) embryonic stem cells, haematopoietic cells or haematopoietic precursor cells derived from the same embryonic stem cell line used for the transplant;
to induce immune tolerance in the recipient of a.) und b.) before receiving the transplant.

Thus, the present invention provides for means and methods for preventing rejection of cell, tissue and/or organ transplants composed of or derived from embryonic stem cell lines. The induction of immune tolerance avoids a life-long immune suppression of the individual by medicaments to avoid rejection of said transplant. This is of particular interest since embryonic stem cells represent a powerful tool for the generation of cell, tissue and/or organ transplants. It is expected that embryonic stem cells will increasingly be used as the source for the generation of bioartificial tissue and/or organs, in gene therapy and in cell transplantation.

The present invention employs the following definitions:
Tolerance: In the present context, tolerance is defined as the durable persistence of foreign cells, tissue, or organs, e.g. of transplants in the absence of a detrimental host immune response, achieved without ongoing therapeutic intervention.
Transplant: Herein, transplant means any cells, tissue or organ transplanted into an individual. In the following, graft has the same meaning as transplant and may be used herein accordingly.

"Embryonic stem cell lines" as used herein comprises established ES-cell lines and newly isolated and cultured ES-cells and ES-cell lines. The distinguishing feature of ES cells from other cells is their capacity to be maintained indefinitely in an undifferentiated state in culture, thus, forming an embryonic cell line of unchanged embryonic cells.

"Derived from embryonic stem cell lines" means that the cells, tissues or organs are generated from cultured embryonic stem cells, i.e. embryonic stem cell lines, via differentiation using suitable differentiating agents, like growth factors, or inhibitors or deactivators for specific molecules, like inhibitors for transcription factors, in order to differentiate the embryonic stem cells into the respective cell types.

The invention is based on the finding that a trophoblast component within the transplanted ES-like cell cultures is critical for induction of mixed chimerism and long-term graft acceptance.

The ES-cell mediated tolerance could allow allotransplantation of ES-cell-derived cells and tissues without any immunosuppression. Said allogeneic tolerance can be achieved in any kind of mammals including humans.

The inventors found that not only ES-like cells but also trophoblasts which may be blastocyste-derived contribute to the induction of immune tolerance. Transplanted ES-like cells are much likely to be the origin of chimeric white blood cells present in the individual after receiving the composition according to the present invention, as it is known that embryonic stem cells are able to differentiate towards haematopoietic lineages. However, the present invention allows transplantation of fully MHC mismatched pure ES-cell lines without any immunosuppression which i.a. resulted in stable mixed chimerism. This is achieved by using not only ES-like cells but also trophoblasts in a pre-treatment step. Since trophoblasts express high levels of FasL as shown by Northern blotting experiments, co-transplantation of these cells may have resulted in depletion of alloreactive T-cell clones thereby preventing rejection of the grafted ES-like cells. The formation of trophoblasts from ES-cells is known in the art.

According to the present invention, a mixture of ES cell-derived trophoblasts and undifferentiated ES cells or ES-cell derived haematopoietic stem cells is used to induce stable haematopoietic chimerism in patients, e.g. with leukemic disorders, a disease today many of patients die before suitable bone marrow transplants are available. In addition, the approach could be used to induce tolerance towards other cell and tissue types generated *in vitro* from the same isogeneic ES-cell line. Such an approach would eliminate the need for pharmacological immunosuppression after transplantation of ES-cell derived grafts or the ethical controversial and cost intensive generation of autologous "custom made" ES-cells by means of therapeutic cloning.

Thus, the invention provides for compositions allowing for the induction of tolerance towards cell preparations which may be in form of cell, tissue or organ transplants derived from an ES-cell line containing on the one hand ES-cells and/or haematopoietic (precursor) cells derived therefrom being identical with the ES-cell used for the generation of the transplant and on the other hand trophoblasts or precursor of trophoblasts and/or placental cells derived from said trophoblasts, said trophoblasts being derived from the identical ES-cell line by differentiation.

The differentiation of the ES-cells to trophoblasts, precursors thereof and/or placental cells may be achieved by using differentiating factors like BMP-4 or e.g. by inhibition or inactivation of transcription factor Oct-4. For example, the trophoblasts may be generated as described in Xu, Ren-He et al., 2002, Nat. Biotechnol., 20(12), 1261. The stage of differentiation may vary and may be even beyond the trophoblasts stage, e.g. placental cells. Hence, the composition according to the present invention contains trophoblasts, precursors thereof and/or placental cells derived from said trophoblasts.

Alternatively, trophoblasts may be substituted by other known types of cells which are able to induce tolerance by inactivating or promoting the deletion of reactive T-cell clones. In this regard, Sertoli cells may be mentioned.

The second type of cells to be used according to the present invention are the embryonic stem cells themselves or haematopoietic stem cells or precursor cells thereof being obtained by differentiation of said embryonic stem cells.

The composition comprising the above types of cells administered in a pre-treatment step to an individual which subsequently undergoes cell, tissue and/or organ transplantation. The graft is derived from the same embryonic stem cell line as the above cell types are.

This means, that the graft to be transplanted into a host may be in the form of an isogeneic cell, tissue and/or organ preparation, e.g. a bioartificial tissue or organ. Said grafts may be obtained by differentiation of embryonic stem cell lines being genetically identical to the embryonic stem cell lines used for the preparation of the above cell types. The differentiation of the cell, tissue and/or organ preparation which depends on the indication and the types of cells needed (e.g. cardiomyocytes, β-cells, hepatocytes neuronal cells, etc.) and tissue or organs composed thereof is timely independent from the differentiation to produce a trophoblasts and haematopoietic stem cells, respectively.

The graft is then transplanted into the recipient whereby the risk of graft versus host reaction is dramatically decreased because of the prior induction of immune tolerance. Using genetically identical, i.e. isogeneic embryonic stem cells for the preparation of the composition to be used in the pre-treatment step and for the generation of the graft allows for an allogeneic transplantation with a decreased risk of graft versus host reaction and, in addition, without the requirement of life-long pharmacological immunesuppression.

The embryonic stem cells to be used according to the present invention may be established ES-cell lines or may be newly isolated primary or cultured ES-cells or ES-cell lines. In a particular embodiment, established ES-cell lines which are known in the art are used for the preparation of the respective components of the composition as well as for the generation of the cell, tissue and/or organ transplant.

Thus, the composition according to the present invention may comprise the trophoblasts, precursors thereof and/or placental cells and the embryonic stem cells, haematopoietic stem cells or precursor cells as a mixture or separately. That is, each type of cells may be present as a single suspension, e.g. in physiological medium. Alternatively, the cells may be present as an admixture of both components.

Further, when the two components of the composition according to the present invention are provided separately, the components may be administered simultaneously, separately or sequentially. Thus, the present invention comprises the use of the components as single preparations or as an admixture thereof. In addition, the components may form a kit comprising at least a single preparation comprising both types of cells or preparations containing the different types of cells separately. Preferably the cells are provided as suspensions, preferably as suspension in a physiological medium.

In addition, the compositions according to the present invention may further comprise pharmaceutically acceptable carrier, additives, diluents and/or additional pharmaceutically active ingredients as known in the art depending on the kind of administration and the indication.

The embryonic stem cells or embryonic stem cell line to be used according to the present invention may further be genetically manipulated. That means, said cells may be obtained by genetic engineering which may comprise introducing additional genes, into the cells, substituting specific genes with modified, e.g. mutated forms thereof or deleting genes or gene fragments. The trophoblasts to be used according to the present invention may be derived from said genetically engineered cells while the cells of the second component, i.e. embryonic stem cells themselves or haematopoietic stem cells or precursor cells being obtained by differentiation of said embryonic stem cells may be derived from the original cell line or may also be derived from the genetically engineered cell line. Of course, it is possible that the trophoblasts component is derived from the original cell line while the ES-cell component may be derived from genetically engineered ES-cell line. In addition, both cell types may be derived from the identical ES-cell line which has been differently genetically engineered. Of course, it is possible to use ES-cells with more than one genetic manipulation as the starting material. For example, the ES-cells used to obtain trophoblasts, etc. contain two additional genes while the ES-cells used to obtain the second component contain only one additional gene or vice versa.

Moreover, the transplant may be derived from genetically engineered ES-cells. The alteration of these cells may be identical or may be different to the ES-cells used to obtain the trophoblasts, etc or the embryonic stem cells, etc. according to the present invention. Depending on the purpose, genetic engineering may be employed. For example, the ES-cells may contain genes which products are helpful for the in vitro generation of said transplant.

In addition the genetic manipulation of the ES-cells may be transiently manipulated.

According to the present invention, induction of immune tolerance can be achieved by using trophoblasts, precursors thereof and/or placental cells alone in the pre-treatment step. Thus, trophoblasts, precursors thereof and/or placental cells derived from an embryonic stem cell line are sufficient to induce immune tolerance towards cell, tissue and/or organ transplantation or a transplant derived from the same embryonic stem cell line. Consequently, the pre-treatment step in a method for inducing immune tolerance in an individual receiving a transplant derived from an embryonic stem cell line according to the present invention comprises administering a composition according to the present invention comprising trophoblasts, precursors thereof and/or placental cells derived from the same embryonic cell line.

The present invention may be used in different fields of medicine and biotechnology, for example, the present invention is beneficial in the field of cell, tissue and/or organ transplantation, in the field of tissue engineering and in gene therapy.

In the field of cell, tissue and/or organ transplantation, the present invention enables the use of allogeneic embryonic stem cells and cell, tissue and/or organs derived therefrom as grafts independent on the MHC pattern of the recipient. Further, no additional immunosuppression is necessary. In addition, it is possible to use genetically engineered cells. Thus, it is possible to induce stable haematopoietic chimerism in patients, e.g. with leukemic disorders. Today, many of those patients die before suitable bone marrow transplants are available. In addition, it is possible to induce tolerance towards other cell and tissue types generated in vitro from the same isogeneic ES-cell.

Another field is the tissue engineering, i.e. the production of bioartificial tissue or organs from embryonic stem cells or cells derived therefrom. The thus generated tissue can be transplanted into the recipient regardless of the MHC pattern and no further immunsuppression is required.

Moreover, the present invention is applicable in the field of gene therapy. In particular, it is possible to establish genetically manipulated embryonic cell lines which may be applied to a recipient for gene therapy. Thus, it is possible to allow for gene therapy regardless of the MHC pattern of the recipient and without the requirement of further immunsuppression treatment.

## Claims

1. Composition comprising
a. trophoblasts, precursor of trophoblasts and/or placental cells derived from trophoblasts, whereby said cells are derived from an embryonic stem cell line by differentiation;
b. embryonic stem cells, haematopoietic cells or haematopoietic precursor cells derived from the same embryonic stem cell line used for the cells in a.).

2. Composition according to claim 1 or claim 2, further containing a pharmaceutically acceptable carrier, additive, diluent and/or additional active ingredient.

3. Composition according to any one of claims 1 to 3, wherein at least one cell type is derived from a genetically engineered embryonic stem cell line.

4. Composition according to claim 1, wherein the cells are suspended in physiologic medium.

5. Use of the components a.) and b.) according to claim 1 for the preparation of a composition for the pre-treatment of individuals receiving cell, tissue and/or organ transplantation in order to induce immune tolerance towards the transplant in the individual.

6. Use according to claim 5, wherein at least one cell type is derived from a genetically engineered embryonic stem cell line.

7. Use of the components a.) and b.) according to claim 1 for the preparation of a composition for the pre-treatment of individuals undergoing gene therapy.

8. Use according to claim 7, wherein at least one cell type is derived from a genetically engineered embryonic stem cell line.

9. Kit for inducing immune tolerance in an individual comprising
a. trophoblasts, precursor of trophoblasts and/or placental cells derived from trophoblasts whereby the above cells are derived from an embryonic stem cell line by differentiation;
b. embryonic stem cells, haematopoietic cells or haematopoietic precursor cells derived from the same embryonic stem cell line used for the cells in a.).

10. Kit according to claim 9, wherein at least one cell type is derived from a genetically engineered embryonic stem cell line.

11. Kit according to claim 9 further comprising instructions for inducing tolerance in an individual.
